# FASCICULE DE BREVET EUROPEEN

(11) **EP 0 774 938 B1**
(45) Date de publication et mention de la délivrance du brevet: **05.01.2000**
(21) Numéro de dépôt: 95927776.5
(22) Date de dépôt: 10.08.1995
(51) Int. Cl.: A61F 2/36

(54) **PROTHESE DE HANCHE**
HÜFTPROTHESE
HIP PROSTHESIS

(30) Priorité: 12.08.1994 FR 9410125
(43) Date de publication de la demande: 28.05.1997
(73) Titulaire: ADVANCED TECHNICAL FABRICATION (Société Anonyme), F-74970 Marignier (FR)
(72) Inventeur: BRESLER, Franck, F-54000 Nancy (FR); CATIER, Philippe, F-35740 Pace (FR); CAUDAL, Philippe, F-83420 La Croix-Valmer (FR); FRANCOIS, Jean-Marie, F-67500 Marienthal (FR); GODEFROY, Jean, F-74130 Ayze (FR); HOROSZOWSKI, Henri, Ramat Chen (IL); MOLE, Daniel, F-54000 Nancy (FR); RIVAT, Paul, F-07130 Saint-Peray (FR)
(74) Mandataire: Poncet, Jean-François
(86) Numéro de dépôt international: FR9501072
(87) Numéro de publication internationale: WO9604868

(56) Documents cités:
- EP-A- 0 038 908
- EP-A- 0 128 036
- EP-A- 0 528 284
- WO-A-91/18560
- DE-A- 3 829 361
- FR-A- 2 618 667
- FR-A- 2 660 855
- FR-A- 2 676 914

## Description

### DOMAINE TECHNIQUE DE L'INVENTION

L'invention concerne les prothèses de hanche comportant une tige conformée pour être introduite dans le canal médullaire d'un fémur, et un col se raccordant à l'extrémité supérieure de la tige pour la relier à une tête sphérique d'articulation.

On a depuis longtemps proposé diverses formes de prothèses de hanche, dans lesquelles les tiges présentent diverses courbures.

On connaît par exemple une prothèse de hanche dont la tige comporte, dans le plan antéro-postérieur, une courbure unique dont le centre est déplacé postérieurement ou antérieurement. Il apparaît qu'une telle forme de prothèse présente un défaut de centrage en zone diaphysaire, pouvant générer des douleurs.

On connaît aussi des prothèses, telles que celles du document FR-A-2 660 855, dont la tige est sensiblement rectiligne dans le plan antéro-postérieur. Ces prothèses présentent également un défaut de centrage pouvant générer des douleurs, et la transmission des contraintes mécaniques sur le fémur est mal répartie.

Le document EP-A-0 038 908 propose une autre forme de prothèse dans laquelle la tige présente, dans la région proximale, une courbure à centre de courbure déplacé antérieurement, tandis que la région distale de la tige présente une courbure à centre de courbure déplacé postérieurement. Cette forme de prothèse est difficile à mettre en place, précisément à cause des courbures, et elle est également difficile à extraire. Le centrage s'avère délicat, et souvent défectueux.

Les documents FR-A-2 618 667 et FR-A-2 676 914 décrivent des prothèses pour fémur gauche dont la tige présente, dans sa zone proximale, une torsion hélicoïdale unique à pas à droite.

Le document WO-A-91 18560 décrit une prothèse pour fémur droit dont la zone proximale présente une torsion hélicoïdale unique à pas à gauche.

Dans ces documents, la torsion hélicoïdale unique va dans le sens de l'antéversion du col.

Par contre, les documents EP-A-0 128 036 et DE-A-38 29 361 décrivent tous deux une prothèse pour fémur gauche dont la partie proximale présente une torsion hélicoïdale unique à pas à gauche. Dans le document EP-A-0 128 036, cette torsion hélicoïdale unique est associée à une légère courbure postérieure de la partie distale de tige, tandis que la partie proximale de tige est sensiblement rectiligne dans le plan antéro-postérieur.

Il apparait que les prothèses décrites dans les documents ci-dessus, avec une torsion hélicoidale unique, ne s'adaptent pas de façon suffisamment efficace dans le canal médullaire du fémur pour obtenir une stabilité à long terme appropriée et une bonne longévité de la prothèse.

### EXPOSE DE L'INVENTION

Le problème proposé par la présente invention est de définir une nouvelle structure de prothèse de hanche, qui, tout en pouvant être scellée par du ciment dans le fémur, puisse être posée sans ciment dans la majorité des cas. Malgré l'absence de ciment, la solidarisation de la prothèse doit être mécaniquement résistante, et l'on doit obtenir une stabilité améliorée de la prothèse.

L'idée consiste alors à :
- répartir de façon optimale sur la partie métaphysaire de prothèse les efforts de flexion et de torsion entre la prothèse et l'os,
- assurer le blocage de la prothèse dans tous les plans de l'espace en zone métaphysaire,
- répartir de manière sensiblement constante l'espace entre la surface de partie métaphysaire de prothèse et la surface correspondante de l'os,
- obtenir un centrage de l'ensemble de la tige dans la cavité qui la reçoit dans le fémur, tant dans le plan frontal que dans le plan antéro-postérieur,
- optimiser le rapport entre la section de partie distale de la tige de prothèse et la section du canal de la diaphyse fémorale, pour éviter les points de contacts douloureux de la tige,
- supprimer la transmission d'efforts par la partie distale de la tige en zone diaphysaire du fémur.

Pour cela, on prévoit une prothèse dont la tige présente une forme différente de celles qui sont connues, certaines caractéristiques de forme de cette tige étant directement contraires à celles habituellement préconisées. Ainsi, l'invention prévoit une prothèse de hanche comportant une tige destinée à être introduite et fixée dans le canal médullaire d'un fémur, un col se raccordant à l'extrémité supérieure de la tige pour la relier à une tête sphérique d'articulation, la tige comprenant une zone proximale à section transversale oblongue s'étendant depuis l'extrémité supérieure de part et d'autre d'un épaulement externe jusqu'à une zone de transition au-dessous de l'épaulement externe suivie d'une zone distale, la tige ayant dans le plan latéral une courbure à centre de courbure déporté vers le plan sagittal, la zone proximale de tige ayant, dans le plan antéro-postérieur, une partie courbe à centre de courbure déplacé vers l'avant ;
la zone proximale à section transversale oblongue comprend deux segments adjacents à torsions hélicoïdales inverses:
   - un segment proximal présentant une torsion hélicoïdale à pas à droite dans le cas d'une prothèse pour fémur droit, et à pas à gauche dans le cas d'une prothèse pour fémur gauche,
   - un segment distal présentant une torsion hélicoïdale à pas à gauche dans le cas d'une prothèse pour fémur droit, et à pas à droite dans le cas d'une prothèse pour fémur gauche.

Il apparaît qu'une telle prothèse peut aisément être introduite dans le canal médullaire, et procure un blocage nettement amélioré en zone métaphysaire.

Selon un mode de réalisation avantageux, le segment proximal et le segment distal de zone proximale à section transversale oblongue se raccordent l'un à l'autre sensiblement dans la zone contenant l'épaulement externe.

De préférence, dans le plan antéro-postérieur, le segment proximal de zone proximale de tige est sensiblement rectiligne, tandis que le segment distal de zone proximale de tige présente une courbure à centre de courbure décalé vers l'avant. Egalement, dans le plan antéro-postérieur, la zone distale de tige est sensiblement rectiligne.

Le col présente avantageusement une antéversion, d'angle égal à 10° environ.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles:
- la figure 1 est une vue en plan d'une prothèse gauche vue d'arrière, associée à neuf coupes transversales successives réalisées le long de la tige de prothèse ;
- la figure 2 est une vue en plan du côté externe de la prothèse gauche de la figure 1 ;
- la figure 3 est une vue d'avant de la prothèse de la figure 1 ;
- la figure 4 illustre schématiquement la face latérale interne de la prothèse de la figure 1 ;
- la figure 5 est une vue schématique en perspective de la prothèse de la figure 1, illustrant la rotation des coupes successives ;
- la figure 6 est une vue schématique de dessus de la prothèse de la figure 1 ;
- la figure 7 est une vue en plan d'une prothèse droite vue d'avant, selon un mode de réalisation préféré de l'invention, associée à huit coupes transversales successives réalisées le long de la tige de prothèse ; et
- la figure 8 est une vue du côté externe de la prothèse de la figure 7, associée aux mêmes coupes transversales successives.

### DESCRIPTION DES MODES DE REALISATION PREFERES

Comme illustré sur les figures, la prothèse de hanche selon l'invention comporte une tige 1 destinée à être introduite et fixée dans le canal médullaire d'un fémur, et un col 2 se raccordant à l'extrémité supérieure 3 de la tige 1 pour la relier à une tête sphérique 4 d' articulation.

La tige 1 présente une forme non linéaire qui varie en section et en orientation en fonction de la position considérée sur sa longueur H.

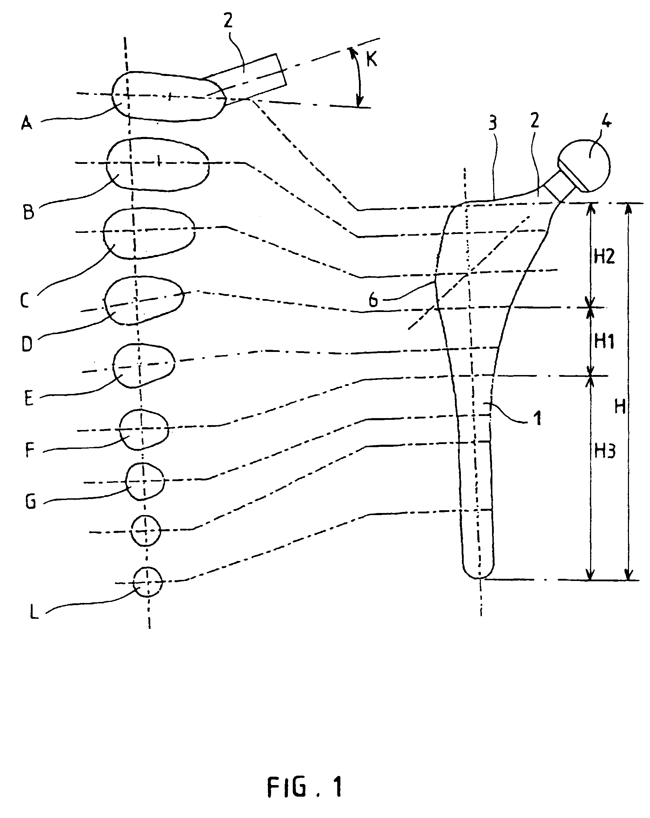

On distingue une zone proximale, proche du col 2, et une zone distale plus éloignée du col 2. La zone distale a une longueur désignée par l'indication H3 sur la figure 1, tandis que la zone proximale est désignée par les références H1 et H2.

La zone proximale H1 + H2 de tige 1 présente une section transversale non circulaire, avantageusement oblongue, illustrée sur la partie gauche des figures 1, 7 et 8. On distingue ainsi sur les figures 1, 7 et 8, en partant de l'extrémité supérieure 3, une section supérieure A, une première section intermédiaire B, une seconde section intermédiaire C, une troisième section intermédiaire D, une quatrième section intermédiaire E et une section de transition F. Ces sections transversales A, B, C, D, E et F présentent une forme allongée, par exemple oblongue, dont les grands axes respectifs pivotent progressivement au fur et à mesure que l'on se déplace le long de l'axe longitudinal de la tige 1. Ce pivotement définit des torsions hélicoïdales, dans la zone proximale H1 + H2 de tige 1.

La zone proximale H1 + H2 s'étend, depuis l'extrémité supérieure 3, de part et d'autre d'un épaulement externe 6, jusqu'à une zone de transition illustrée par la section de transition F, au-dessous de l'épaulement externe 6. La zone proximale est suivie de la zone distale H3, dont les sections transversales telles que les sections G et L sont généralement circulaires.

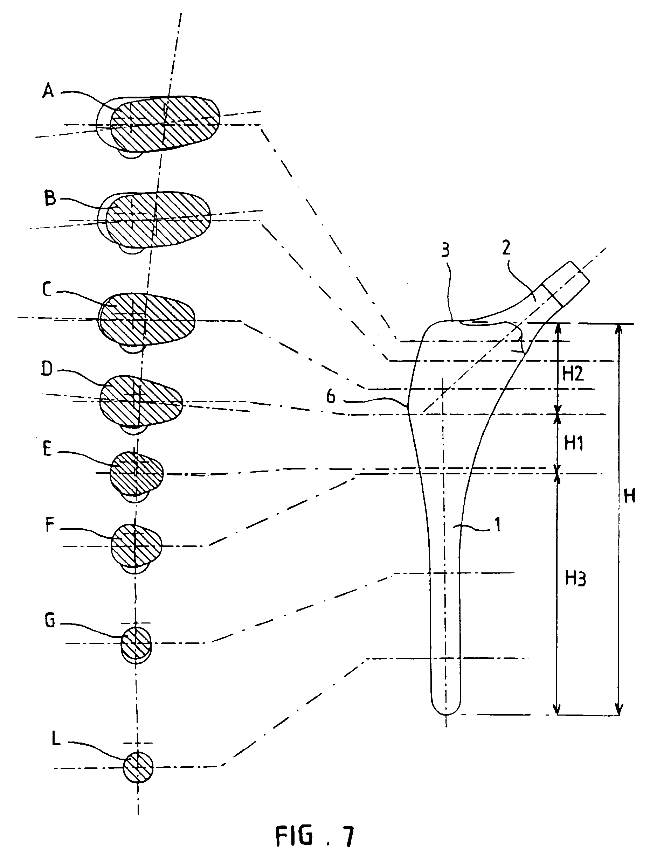

Comme illustré sur les figures 1 et 7, en partie gauche, la tige 1 présente, dans le plan latéral, une courbure à centre de courbure déportée vers le plan sagittal.

La zone proximale H1 + H2 de tige 1 comprend un segment proximal H2, proche du col 2, et un segment distal H1 plus éloigné du col 2. Le segment proximal H2 présente une torsion hélicoïdale à pas à droite dans le cas d'une prothèse pour fémur droit, et à pas à gauche dans le cas d'une prothèse pour fémur gauche. Ainsi, sur la figure 1, les sections transversales successives A, B, C et D illustrent la torsion hélicoïdale à pas à gauche pour la prothèse gauche, tandis que, sur la figure 7, les mêmes sections transversales A, B, C et D illustrent la torsion hélicoïdale à pas à droite pour la prothèse droite.

Le segment distal H1 présente une torsion hélicoïdale à pas à gauche dans le cas d'une prothèse pour fémur droit, et à pas à droite dans le cas d'une prothèse pour fémur gauche. Ainsi, sur la figure 1, les sections transversales successives D, E et F illustrent la torsion hélicoïdale à pas à droite pour une prothèse gauche dans le segment distal H1, tandis que, sur la figure 7, les mêmes sections D, E et F illustrent la torsion hélicoïdale à pas à gauche pour une prothèse droite dans le segment distal H1.

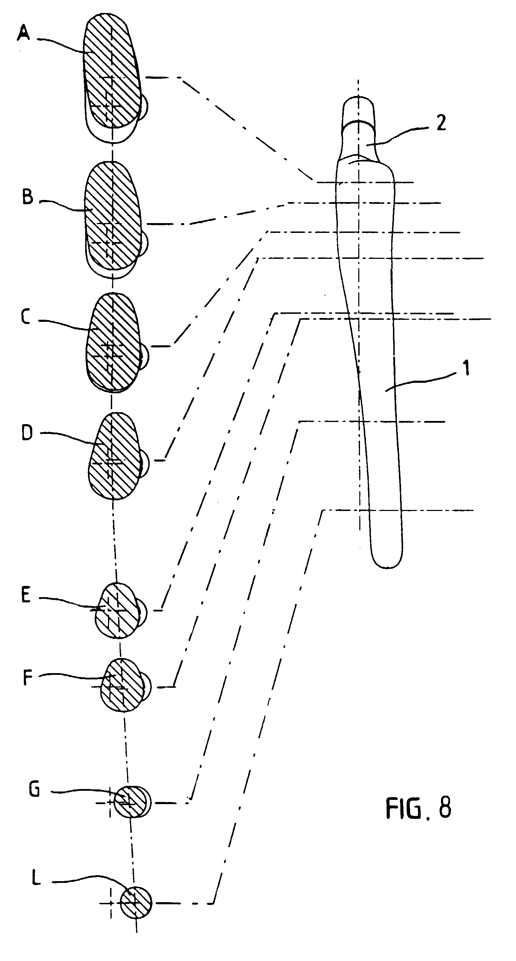

Dans le mode de réalisation illustré sur les figures 7 et 8, le segment proximal H2 et le segment distal H1 de zone proximale à section transversale oblongue se raccordent avantageusement l'un à l'autre sensiblement dans la zone contenant l'épaulement externe 6.

Dans la réalisation illustrée sur les figures 7 et 8, le segment proximal H2 présente une torsion hélicoïdale dont l'angle de torsion global est compris entre 9° et 13° environ, avantageusement égal à 11° environ. De même, le segment distal H1 présente une torsion hélicoïdale dont l'angle de torsion global est compris entre 3° et 6°, avantageusement égal à 5° environ.

Le segment distal H1 est légèrement plus court que le segment proximal H2.

La zone proximale H1 + H2 ou métaphysaire. comportant la double torsion hélicoïdale précédemment définie, peut avoir une longueur H1 + H2 comprise entre la moitié et le tiers de la longueur totale H de la tige 1, dans le cas d'une prothèse standard. Dans le cas d'une tige plus longue, par exemple pour une reprise, cette proportion varie en fonction de la longueur choisie de la tige.

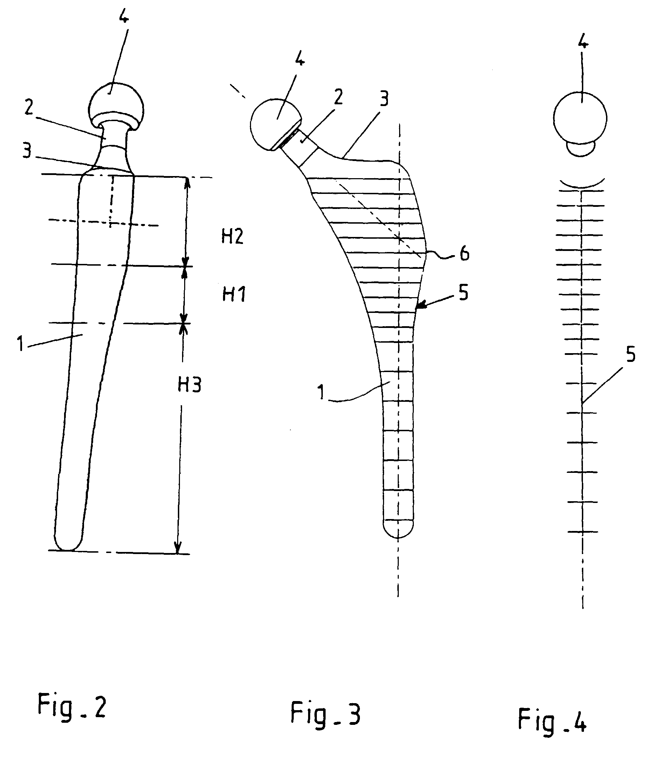

La zone proximale H1 + H2 ou métaphysaire qui présente la double torsion hélicoïdale peut être généralement rectiligne dans le plan antéro-postérieur, courue illustré dans le mode de réalisation de la figure 2. Cette zone métaphysaire présente naturellement une courbure vers l'intérieur dans le plan transversal, courue illustré sur la figure 1.

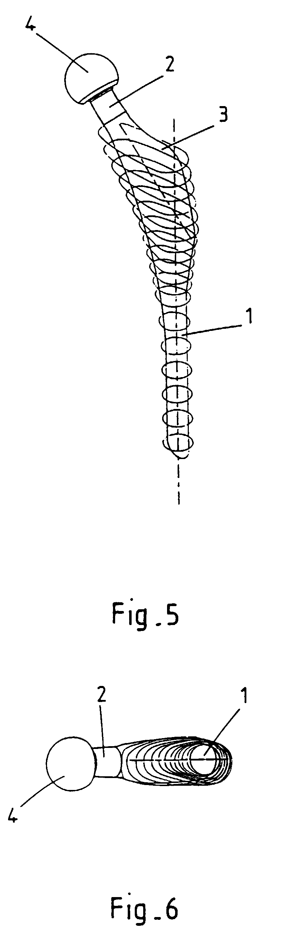

Dans le mode de réalisation représenté sur les figures 1 à 6, les zones métaphysaire et diaphysaire sont généralement rectilignes. Cela apparaît notamment sur la face latérale interne de prothèse telle qu'illustrée sur la figure 4 : la ligne médiane 5 de face latérale interne est sensiblement contenue dans le plan transversal, de sorte qu'elle paraît rectiligne sur la figure.

Dans le mode de réalisation préféré, illustré sur les figures 7 et 8, dans le plan antéro-postérieur, le segment proximal H2 de zone proximale de tige 1 est sensiblement rectiligne, tandis que le segment distal H1 de zone proximale présente une courbure à centre de courbure décalé vers l'avant. La courbure est mieux visible sur la figure 8, en partie gauche. Dans ce même plan antéro-postérieur, la zone distale H3 de tige 1 est sensiblement rectiligne. On a pu constater que ce mode de réalisation assure une stabilité améliorée et un meilleur centrage de la prothèse dans le fémur. Les dessins des figures 7 et 8 représentent assez fidèlement les proportions d'une prothèse moyenne selon l'invention, et les dimensions à une échelle d'environ 0,7.

Le col 2 présente une antéversion, illustrée sur la coupe A de la figure 1. L'antéversion est définie par l'angle K entre le plan transversal et l'axe du col 2. L'angle K d'antéversion du col 2 est avantageusement égal à 10° environ.

Grâce à la forme d'une telle prothèse, on peut éviter dans la plupart des cas, d'avoir recours à un ciment pour assurer le scellement de la tige de prothèse dans le canal médullaire.

On évite ainsi les inconvénients liés à la présence de ciment :
augmentation du temps de pose et de la morbidité opératoire, production d'une réaction exothermique qui provoque entre autres une nécrose de l'os au contact du ciment lors de la pose, développement de tissus fibreux se formant entre le ciment et l'os, usure anormale de la prothèse par suite des déplacements de débris de ciment, qui en réduisent la longévité.

La tonne en double torsion hélicoïdale de la zone proximale ou métaphysaire procure un solide blocage antéro-postérieur de la prothèse.

Pour augmenter la stabilité de la prothèse, on peut prévoir des reliefs en forme de vagues dans la partie proximale ou métaphysaire. Ces reliefs augmentent la surface de contact os-implant, sans toutefois s'opposer â son extraction éventuelle par le chirurgien. Par ces reliefs, on crée également des parties de surface de contact perpendiculaires à l'axe de l'os, pour reprendre les efforts axiaux. Ces reliefs peuvent prendre la tonne de nervures périphériques ondulées.

La présente invention n'est pas limitée aux modes de réalisation qui ont été explicitement décrits, mais elle en inclut les diverses variantes et généralisations contenues dans le domaine des revendications ci-après.

## Revendications

1. Prothèse de hanche comportant une tige (1) destinée à être introduite et fixée dans le canal médullaire d'un fémur, un col (2) se raccordant à l'extrémité supérieure (3) de la tige (1) pour la relier à une tête sphérique (4) d'articulation, la tige (1) comprenant une zone proximale (H1 + H2) à section transversale oblongue s'étendant depuis l'extrémité supérieure (3) de part et d'autre d'un épaulement externe (6) jusqu'à une zone de transition (F) au-dessous de l'épaulement externe (6) et suivie d'une zone distale (H3), la tige ayant dans le plan latéral une courbure à centre de courbure déporté vers le plan sagittal, la zone proximale (H1 + H2) de tige ayant, dans le plan antéro-postérieur, une partie courbe à centre de courbure déplacé vers l'avant,
caractérisée en ce que la zone proximale (H1 + H2) à section transversale oblongue comprend deux segments adjacents à torsions hélicoïdales inverses:
- un segment proximal (H2) présentant une torsion hélicoïdale à pas à droite dans le cas d'une prothèse pour fémur droit, et à pas à gauche dans le cas d'une prothèse pour fémur gauche,
- un segment distal (H1) présentant une torsion hélicoïdale à pas à gauche dans le cas d'une prothèse pour fémur droit, et à pas à droite dans le cas d'une prothèse pour fémur gauche.

2. Prothèse de hanche selon la revendication 1, caractérisée en ce que le segment proximal (H2) et le segment distal (H1) de zone proximale à section transversale oblongue se raccordent l'un à l'autre sensiblement dans la zone contenant l'épaulement externe (6).

3. Prothèse de hanche selon l'une des revendications 1 ou 2, caractérisée en ce que le segment proximal (H2) présente une torsion hélicoïdale d'angle compris entre 9° et 13° environ.

4. Prothèse de hanche selon la revendication 3, caractérisée en ce que le segment proximal (H2) présente une torsion hélicoïdale d'angle égal à 11° environ.

5. Prothèse de hanche selon l'une quelconque des revendications 1 à 4, caractérisée en ce que le segment distal (H1) présente une torsion hélicoïdale d'angle compris entre 3° et 6° environ.

6. Prothèse de hanche selon la revendication 5, caractérisée en ce que le segment distal (H1) présente une torsion hélicoïdale d'angle égal à 5° environ.

7. Prothèse de hanche selon l'une quelconque des revendications 1 à 6, caractérisée en ce que le segment distal (H1) est légèrement plus court que le segment proximal (H2).

8. Prothèse de hanche selon l'une quelconque des revendications 1 à 7, caractérisée en ce que, dans le plan antéro-postérieur, le segment proximal (H2) de zone proximale de tige (1) est sensiblement rectiligne, tandis que le segment distal (H1) de zone proximale de tige (1) présente une courbure à centre de courbure décalé vers l'avant.

9. Prothèse de hanche selon l'une quelconque des revendications 1 à 8, caractérisée en ce que, dans le plan antéro-postérieur, la zone distale (H3) de tige (1) est sensiblement rectiligne.

10. Prothèse de hanche selon l'une quelconque des revendications 1 à 9, caractérisée en ce que le col (2) présente une antéversion d'environ 10°.

## Claims

1. Hip prosthesis including a rod (1) adapted to be inserted and fixed into the medullary canal of a femur, a neck (2) joined to the upper end (3) of the rod (1) to join it to a spherical joint head (4), the rod (1) having an oblong transverse section proximal area (H1 + H2) extending from the upper end (3) on either side of an external shoulder (6) to a transition area (F) below the external shoulder (6) and followed by a distal area (H3), the rod being curved in the lateral plane with a centre of curvature offset towards the sagittal plane, the proximal area (H1 + H2) of the rod having a curved portion, in the anterior-posterior plane, with the centre of curvature offset towards the front,
characterised in that the oblong transverse section proximal area (H1 + H2) comprises two adjacent segments with opposite spiral twists :
- a proximal segment (H2) having a righthanded spiral twist in a prosthesis for a right femur, and a lefthanded spiral twist in a prosthesis for a left femur,
- a distal segment (H1) having a lefthanded spiral twist in a prosthesis for a right femur, and a righthanded spiral twist in a prosthesis for a left femur.

2. Hip prosthesis according to claim 1, characterised in that the proximal segment (H2) and the distal segment (H1) of the oblong transverse section proximal area are joined together substantially in the area containing the external shoulder (6).

3. Hip prosthesis according to claim 1 or 2, characterised in that the proximal segment (H2) has a spiral twist with an angle between about 9° and about 13°.

4. Hip prosthesis according to claim 3, characterised in that the proximal segment (H2) has a spiral twist with an angle equal to about 11°.

5. Hip prosthesis according to any one of claims 1 to 4, characterised in that the distal segment (H1) has a spiral twist with an angle between about 3° and about 6°.

6. Hip prosthesis according to claim 5, characterised in that the distal segment (H1) has a spiral twist with an angle equal to about 5°.

7. Hip prosthesis according to any one of claims 1 to 6, characterised in that the distal segment (H1) is slightly shorter than the proximal segment (H2).

8. Hip prosthesis according to any one of claims 1 to 7, characterised in that the proximal segment (H2) of the proximal area of the rod (1) is substantially straight, in the anterior-posterior plane, whereas the distal segment (H1) of the proximal area of the rod (1) is curved with the centre of curvature offset towards the front.

9. Hip prosthesis according to any one of claims 1 to 8, characterised in that the distal area (H3) of the rod (1) is substantially straight in the anterior-posterior plane.

10. Hip prosthesis according to any one of claims 1 to 9, characterised in that the neck (2) has an anteversion of about 10°.

## Patentansprüche

1. Hüftprothese mit einem Schaft (1), der zum Einführen und Befestigen in einer Markröhre eines Oberschenkelknochens vorgesehen ist, einem Hals (2), der mit einem oberen Ende (3) des Schaftes (1) verbunden ist, um diesen mit einem Kugelgelenkkopf (4) zu verbinden, wobei der Schaft (1) einen proximalen Bereich (H1 + H2) mit einem länglichen Querschnitt aufweist, der sich von dem oberen Ende (3) auf beiden Seiten einer äußeren Schulter (6) bis in einen Übergangsbereich (F) unterhalb der äußeren Schulter (6) erstreckt und von einem distalen Bereich (H3) gefolgt ist, wobei der Schaft an der seitlichen Fläche eine Krümmung mit einem Krümmungsmittelpunkt aufweist, der in Richtung der Sagittalebene verschoben ist, wobei der proximale Bereich (H1 + H2) des Schaftes in der antero-posterioren Ebene ein gekrümmtes Stück mit einem Krümmungsmittelpunkt aufweist, der nach vorne versetzt ist,
dadurch gekennzeichnet, daß der proximale Bereich (H1 + H2), der einen länglichen Querschnitt hat, zwei benachbarte Abschnitte mit entgegengesetzter schraubenförmiger Torsion aufweist:
- einen proximalen Abschnitt (H2), der eine rechtsgerichtete schrauhenförmige Torsion aufweist, für den Fall einer Prothese für den rechten Oberschenkelknochen, und eine linksgerichtete, für den Fall einer Prothese für den linken Oberschenkelknochen,
- einen distalen Abschnitt (H1), der eine linksgerichtete schraubenförmige Torsion aufweist, für den Fall einer Prothese für den rechten Oberschenkelknochen, und eine rechtsgerichtete, für den Fall einer Prothese für den linken Oberschenkelknochen.

2. Hüftprothese nach Anspruch 1, dadurch gekennzeichnet, daß der proximale Abschnitt (H2) und der distale Abschnitt (H1) des proximalen Bereiches mit länglichem Querschnitt ungefähr in dem Bereich miteinander verbunden sind, der die äußere Schulter (6) aufweist.

3. Hüftprothese nach einem der Ansprüche 1 oder 2, dadurch gekennzeichnet, daß der proximale Abschnitt (H2) eine schraubenförmige Torsion mit einem Winkel zwischen etwa 9° und 13° aufweist.

4. Hüftprothese nach Anspruch 3, dadurch gekennzeichnet, daß der proximale Abschnitt (H2) eine schraubenförmige Torsion mit einem Winkel aufweist, der etwa gleich 11° ist.

5. Hüftprothese nach einem der Ansprüche 1 bis 4, dadurch gekennzeichnet, daß der distale Abschnitt (H1) eine schraubenförmige Torsion mit einem Winkel zwischen etwa 3° und 6° aufweist.

6. Hüftprothese nach Anspruch 5, dadurch gekennzeichnet, daß der distale Abschnitt (H1) eine schraubenförmige Torsion mit einem Winkel aufweist, der etwa gleich 5° ist.

7. Hüftprothese nach einem der Ansprüche 1 bis 6, dadurch gekennzeichnet, daß der distale Abschnitt (H1) etwas kürzer als der proximale Abschnitt (H2) ist.

8. Hüftprothese nach einem der Ansprüche 1 bis 7, dadurch gekennzeichnet, daß in der antero-posterioren Ebene der proximale Abschnitt (H2) des proximalen Bereiches des Schaftes (1) ungefähr gerade ist, wohingegen der distale Abschnitt (H1) des proximalen Bereiches des Schaftes (1) eine Krümmung mit einem Krümmungsmittelpunkt aufweist, der nach vorne verschoben ist.

9. Hüftprothese nach einem der Ansprüche 1 bis 8, dadurch gekennzeichnet, daß in der antero-posterioren Ebene der distale Bereich (H3) des Schaftes (1) ungefähr gerade ist.

10. Hüftprothese nach einem der Ansprüche 1 bis 9, dadurch gekennzeichnet, daß der Hals (2) eine Neigung von etwa 10° hat.
